# EUROPEAN PATENT APPLICATION

(11) **EP 1 615 032 A1**
(43) Date of publication of application: **11.01.2006**
(21) Application number: 04715530.4
(22) Date of filing: 27.02.2004
(51) Int. Cl.: G01N 33/50, G01N 33/15, G01N 33/53, G01N 33/566

(54) **METHOD OF DETECTING ONSET RISK OF ENCEPHALITIS OR ENCEPHALOPATHY**

(30) Priority: 27.02.2003 JP 2003051678
(71) Applicant: Kido, Hiroshi, Tokushima-shi, Tokushima 770-8503 (JP)
(72) Inventor: Kido, Hiroshi, Tokushima-shi, Tokushima 770-8503 (JP)
(74) Representative: HOFFMANN EITLE
(86) International application number: PCT/JP2004/002430
(87) International publication number: WO 2004/077055

(57) **Abstract**

The invention provides a method of detecting the onset of encephalitis/encephalopathy and the risk thereof; a method of judging as to whether the administration or application of a drug is safe or not (as to whether it has the risk of inducing influenzal encephalitis/encephalopathy or not); and a method of screening for drugs utilizable in the treatment of encephalitis/encephalopathy. The detecting method comprising preparing a voltage-dependent anion channel (VDAC)-containing sample derived from a patient suspected of the onset of encephalitis/encephalopathy, measuring the level of VDAC expression in the sample, and detecting the onset of encephalitis/encephalopathy and the onset sensitivity (onset risk) using the increase in said expression level as an indicator.

## Description

### TECHNICAL FIELD

The present invention relates to a method of detecting (determining) the onset risk of encephalitis or encephalopathy, a method of testing a drug as to whether the drug involves the risk of inducing encephalitis or encephalopathy, and a method of screening for candidate drugs for the treatment of encephalitis or encephalopathy.

### BACKGROUND ART

In recent years, the number of reports about encephalitis or encephalopathy resulting from or following influenza virus infection has been increasing. Such encephalitis/encephalopathy is a disease whose predominant symptoms include rapid manifestations of consciousness disorder and convulsion, in particular a rapid manifestation of brain edema which is often fatal. This encephalitis/encephalopathy has become a social problem because of the high mortality therefrom and the high incidence of sequela thereof.

Influenza infection in young children is often accompanied by such central nervous system symptoms as consciousness disorder, febrile convulsion, prolonged convulsion and vomiting and, in some instances, rapid progress and death may result. In some of such patients, encephalopathy follows the taking of an antipyretic such as Aspirin™ (acetylsalicylic acid) or diclofenac sodium and, in such cases, the disease is referred to as Reye's syndrome. In other cases in which influenza virus infection is involved but the cause is unknown, the particular term influenzal encephalitis/encephalopathy is used in Japan. The pathology and the mechanisms of the onset of this influenzal encephalitis/encephalopathy are unknown, and this has no clear definition as a disease. The difference between the influenzal encephalitis/encephalopathy and the above-mentioned Reye's syndrome or acute necrotizing encephalopathy (ANE) is not distinct (cf. Takehiro Togashi, "Influenzal encephalitis and encephalopathy", Nippon Rinsho (Japanese Journal of Clinical Medicine), Vol. 58, No. 11, pp. 87-92, 2002; and Tsuneo Morishima, "Influenzal encephalitis and encephalopathy: Pathology and strategy", Sogo Rinsho (Clinic All-Around), Vol. 49, No. 2, pp.300-305, 2000).

It is known that, in infants with congenital fatty acid metabolism disorder, in particular with CPT II (carnitine palmitoyltransferase II) deficiency or GA2 (glutaric aciduria type 2) frequently found in Japan and with MCAD (medium-chain acyl-CoA dehydrogenase) deficiency frequently found in Europe and America, encephalopathy manifests itself as Reye's syndrome-like illness without influenza infection (cf. Tamoki, Y., et al., "A survey of Japanese patients with mitochondrial fatty acid β-oxidation and related disorders as detected from 1985 to 2000", Brain & Development, 24 (2002) 675-680).

The mechanisms of the onsets of brain edema and cerebral function disorder in such diseases (Reye's syndrome, influenzal encephalitis/encephalopathy, Reye's syndrome-like illness in congenital fatty acid metabolism disorder, etc.) is unknown even today and, under the existing circumstances, no methods have been established as yet for the accurate differential diagnoses, treatment and prevention of such diseases. It is earnestly desired in the art that such a diagnostic method and so forth be established.

The present inventors have already revealed the occurrence of miniplasmin as a factor essential for the viral multiplication cycle in influenza virus infection (cf. Murakami, M., et al., "Mini-plasmin found in the epithelial cells of bronchioles triggers infection by broad-spectrum influenza A viruses and sendai virus." Eur. J. Biochem. 268 (10), 2847-2855, 2001).

The above-mentioned miniplasmin is known as a factor perturbing the blood-brain barrier (cf. Nagy, Z., et al., "Perturbation of integrity of the blood-brain barrier by fibrolytic enzymes", Blood Coagulation & Fibrinolysis, 9 (6), 471-478, 1998).

In influenza infection-induced encephalitis/encephalopathy, miniplasmin disturbs the blood-brain barrier and rapidly induces brain edema. In some instances, it further induces viral replication in cerebral blood vessel endothelial cells. Further, increased levels of miniplasmin are found at the sites coinciding with the sites of blood-brain barrier disorder and viral replication (cf. Hiroshi Kido et al., "Biogenic proteases and inhibitors inhibiting the infectivity of influenza viruses and the aggravation of the infections", Molecular Medicine, Vol. 39, No. 1, pp. 48-53, 2002).

In connection with Reye's syndrome, a report has been made also from the viewpoint of inhibition of fatty acid β-oxidation (cf. Trauner, D. A., et al., "Inhibition of fatty acid beta oxidation by influenza B virus and salicylic acid in mice: Implications for Reye's syndrome", Neurology, 38, 239-241, 1988).

### DISCLOSURE OF INVENTION

A first object of the present invention is to reveal the pathology, onset factor(s) and/or onset mechanisms of influenzal encephalitis/encephalopathy. More particularly, this first object of the invention is to clarify the onset factors and/or onset mechanisms of various kinds of encephalitis/encephalopathy, including influenzal encephalitis/encephalopathy and Reye's syndrome so far undistinguishable from the influenzal one.

A second object of the invention is to establish methods for the accurate diagnosis, treatment and prevention, among others, of encephalitis/encephalopathy.

A third object of the invention is to provide a method of detecting (determining) the onset and onset risk of encephalitis/encephalopathy.

A fourth object of the invention is to provide a method of determining whether any of various drugs such as antipyretics and analgesics involves a risk of causing the onset of encephalitis/encephalopathy or not, namely a method of testing any of various drugs for the safety from the onset of encephalitis/encephalopathy.

A fifth object of the invention is to provide a method of screening for candidate drugs for the treatment of encephalitis/encephalopathy.

A further object of the invention is to provide various reagents for carrying out the various methods mentioned above.

The inventors have made intensive investigations in an attempt to elucidate the onset factors, onset sensitive factors and/or onset mechanisms of influenzal encephalitis/encephalopathy. In the course of the investigations, they revealed that, in the brain of an influenzal encephalitis/encephalopathy model mouse, miniplasmin produced in an organ affected by inflammation, for example in a lung affected by pneumonia, becomes accumulated, through the blood, in the cerebral blood vessel endothelium and, as a result, it destructs the blood-brain barrier or further allows viral replication localized in vascular endothelial cells.

As a result of continued studies, the inventors found that, in the influenzal encephalitis/encephalopathy model mouse brain, the channel protein VDAC (voltage dependent anion channel) localized in the cerebral blood vessel endothelial cell membrane or mitochondrial outer membrane functions as a miniplasmin, plasmin or plasminogen receptor, that an increased level of expression of said VDAC results in increased levels of miniplasmin, plasmin and plasminogen in the vascular endothelium, and that, accordingly, the VDAC is a direct factor leading to the onset of encephalitis/encephalopathy.

More particularly, the inventors revealed that when the level of expression of the VDAC or the gene coding therefor increases, a condition highly sensitive to brain edema results and, when influenza virus infection or some other inflammation, for instance, additionally acts as a trigger, an increase in permeability of the blood-brain barrier and brain edema as well as malfunction of neurons are induced, leading to the onset of encephalitis/encephalopathy. The mechanisms of the onset of encephalitis/encephalopathy as thus revealed are more specifically as follows.

Thus, the above-mentioned condition highly sensitive to brain edema is induced by various factors below or other unknown factors. The factors are, for example, by (1) influenza virus infection, or some other viral infection, for example RS virus infection, adenovirus infection, measles virus infection, poliovirus infection or malarial infection, (2) taking of an antipyretic such as aspirin, diclofenac sodium or mefenamic acid, (3) a disorder in metabolism of long-chain and medium-chain fatty acids in mitochondria as resulting from a congenital metabolic disorder, for instance, or (4) a disorder in fatty acid metabolism in mitochondria as caused by a decrease in blood cartinine level as induced by a drug or artificial dialysis. In this condition highly sensitive to brain edema, the expression of the VDAC is also at a high level. When such a condition under an increased VDAC expression level is accompanied by the infection with such a pathogen as an influenza virus species, the VDAC expression is still more accelerated or the production of miniplasmin or plasmin is promoted by various inflammatory reactions within the body, among others, and the miniplasmin or plasmin is accumulated in the cerebral blood vessel endothelium. Encephalitis/encephalopathy thus manifests itself.

The present invention has been accomplished as a result of further investigations based on the novel finding that an increased level of VDAC expression is a direct factor causing encephalitis/encephalopathy, as mentioned above.

The invention provides the methods and agent defined below under Items 1-11.
Item 1. A method of detecting encephalitis/encephalopathy comprising preparing a VDAC-containing sample derived from a patient suspected of the onset of encephalitis/encephalopathy, measuring the level of VDAC expression in the sample, and detecting the onset of encephalitis/encephalopathy and the onset sensitivity (onset risk) using the increase in said expression level as an indicator.
Item 2. A method as defined in Item 1, wherein the encephalitis/encephalopathy is influenzal encephalitis/encephalopathy.
Item 3. A method as defined in Item 1 or 2, wherein the measurement of the VDAC expression level is carried out in the manner of measurement of the VDAC gene transcript.
Item 4. A method as defined in Item 1 or 2, wherein the measurement of the VDAC expression level is carried out in the manner of measurement of the VDAC protein.
Item 5. A method defined in any of Items 1-4, wherein the VDAC comprises at least one isoform selected from the group consisting of VDAC-1, VDAC-2 and VDAC-3.
Item 6. A method of determining the onset risk of encephalitis/encephalopathy as entailed by a drug which comprises measuring the VDAC expression level in VDAC-expressing cells in a system in which said drug is present, comparing said expression level with the VDAC expression level in the VDAC-expressing cells in a system in which said drug is absent, and determining the onset risk of encephalitis/encephalopathy as incurred by the drug using the difference in expression level between both as an indicator.
Item 7. A method of determining the onset risk entailed by a drug as defined in Item 6, wherein the VDAC-expressing cell system in which a drug is present is formed by administration of the drug to a test animal.
Item 8. A method of determining the onset risk entailed by a drug as defined in Item 6, wherein the VDAC-expressing cell system in which a drug is present is formed by addition of the drug to a VDAC-expressing cell culture fluid.
Item 9. A method of screening for candidate drugs for the treatment of encephalitis/encephalopathy comprising measuring the VDAC expression level in VDAC-expressing cells in a system in which a test substance is present, comparing the thus-measured expression level with the VDAC expression level in the VDAC-expressing cells in a system in which said test substance is absent, which level serves as a reference value, and selecting the test substance as a candidate drug when it is effective in lowering the expression level as compared with the reference value.
Item 10. A method of screening candidate drugs capable of inhibiting the binding of miniplasmin, plasmin or plasminogen to the VDAC as a receptor, which method comprises measuring the level of binding of miniplasmin, plasmin or plasminogen to the VDAC protein in a system in which a test substance is present, comparing the thus-measured binding level with the level of binding of miniplasmin, plasmin or plasminogen to the VDAC protein in a system in which said test substance is absent, which level serves as a reference value, and selecting the test substance as a candidate drug when it is effective in lowering the level of binding as compared with the reference value.
Item 11. An agent for the treatment of encephalitis/encephalopathy, namely an agent for suppressing the production of miniplasmin and plasmin which bind to the VDAC protein and thereby inhibiting the onset of encephalitis/encephalopathy, as selected by the method defined in Item 9 or 10.

Representation of amino acids, peptides, nucleotide sequences, nucleic acids and so forth by abbreviations in the specification is in conformity with the rules recommended by the IUPAC-IUB "IUPAC-IUB Communication on Biological Nomenclature, Eur. J. Biochem., 138:9 (1989)" or the "Guideline for drafting patent specifications and so forth relative to nucleotide sequences and/or amino acid sequences" (edited by the Japanese Patent Office) and the conventions relating to the use of codes or symbols in the relevant field of art.

The term "VDAC" as used in the specification collectively includes, within the meaning thereof the isoforms of VDAC, namely VDAC-1, -2 and -3, or the VDAC gene and VDAC protein and so forth, without making any distinction among them. The VDAC itself is already known, and the nucleotide sequences of human VDAC-1, -2 and -3, for instance, can be obtained from the information from the National Center for Biotechnology Information (NCBI) and are referred to as "NM003374", "NM003375" and "NM005662", respectively. The nucleotide sequences of murine VDAC-1, -2 and - 3 in encephalitis model mice used in the example section to be described later are also available in the same manner and are respectively referred to as "NM011694", "NM011695" and "NM011696".

The term "influenzal encephalitis/encephalopathy" is used in the specification to refer to a disease caused by influenza virus infection and involving the onsets of pyrexia, spasm and early consciousness disorder, accompanied in some instances by brain edema and bilateral thalamic lesions revealed upon CT. Said influenzal encephalitis/encephalopathy includes all types of encephalitis/encephalopathy accompanying influenza infection and showing a raised level of VDAC expression as an onset factor. In some instances, said influenzal encephalitis/encephalopathy also includes such diseases so far generally defined as Reye's syndrome, acute necrotizing encephalopathy and HSES (hemorrhagic shock and encephalopathy syndrome). Furthermore, such types of encephalitis/encephalopathy that do not conform to those diseases mentioned above but accompanies influenza infection and an increased VDAC expression level as the onset mechanisms are also included therein.

The "encephalitis/encephalopathy" so referred to herein includes not only the above-mentioned influenzal encephalitis/encephalopathy but also various types of encephalitis/encephalopathy in which an increased level of VDAC expression is observed as an onset factor. Thus, irrespective of the cause thereof, even those types of encephalitis/encephalopathy which have so far been classified as Reye's syndrome and so forth are to be included under the encephalitis/encephalopathy as defined herein when an increased VDAC expression level is observed as an onset factor.

In accordance with the present invention, the onset factor(s) and/or onset mechanisms of various types of encephalitis/encephalopathy, typically influenzal encephalitis/encephalopathy, can be elucidated, and methods for exact diagnosis, treatment and prevention, among others, of such encephalitis/encephalopathy can be established. In accordance with the invention, it is also possible to provide a method of determining the onset risk of encephalitis/encephalopathy as incurred by a drug (method of testing the drug), a method of screening for candidate drugs for the treatment of encephalitis/encephalopathy, and various reagents for carrying out these methods.

### BRIEF DESCRIPTION OF THE DRAWINGS

Fig. 1 is a comparative representation of the results of PCR measurements of the numbers of copies of the influenza virus genome in the brain, lung, liver and blood of WT and JVS mice.
Fig. 2 is a representation of the results of determination of the levels of binding of plasminogen, miniplasmin and microplasmin to VDAC.
Fig. 3 is a representation of the results of RT-PCR analysis which reveal increases in VDAC-1 expression level in the brain of WT mice as caused by various onset-inducing factors, typically influenza infection.
Fig. 4 is a comparative graphic representation of the results of real time PCR measurements of the levels of expression of the VDAC-1 mRNA in the brain and lung of WT mice.
Fig. 5 is a comparative graphic representation of the results of real time PCR measurements of the levels of expression of the VDAC-2 mRNA in the brain and lung of WT mice.
Fig. 6 is a comparative graphic representation of the results of real time PCR measurements of the levels of expression of the VDAC-3 mRNA in the brain and lung of WT mice.
Fig. 7 is a photomicrograph obtained in immunohistochemical analysis and showing the expression of the VDAC protein in the brain of a JVS mouse.
Fig. 8 is a photomicrograph obtained in immunohistochemical analysis and showing the expression of the VDAC protein in the brain of a JVS mouse infected with influenza.

### BEST MODES FOR CARYYING OUT THE INVENTION

In the following, the method of diagnosing encephalitis/encephalopathy, the method of determining the safety of a drug and the method of screening for candidate drugs, each in accordance with the present invention, are described one by one in detail.

### (1) Method of detecting (diagnosing) encephalitis/encephalopathy

In carrying out the method of the invention, a VDAC-containing sample derived from a patient suspected of the onset of encephalitis/encephalopathy is first prepared. The sample is not particularly restricted provided that it contains the transcript or translation product (VDAC protein) of the VDAC gene, which is the measurement target. Examples of the sample for transcript assaying are various biological samples containing tissue cells and excised tissues derived from peripheral blood, lymph nodes, cerebrospinal fluid, thoracic cavity fluid, abdominal cavity fluid, washings produced on the occasion of operation, and so forth, or cells of such a tissue as hair. Examples of the sample for translation product assaying are, in addition to the same tissue cells as mentioned above, various body fluids derived from them. These samples can be collected or prepared in the conventional manner.

The measurement of the VDAC expression level in the sample can be made either in the manner of measurement of the VDAC gene expression level in the sample (measurement of the transcript) or in the manner of measurement of the level of expression of the VDAC protein which is the product of expression of that gene (measurement of the translation product). In either case, the measurement can be carried out by various methods commonly known in the art. The VDAC to be measured may be any of the isoforms VDAC-1, -2 and -3 or may be a mixture at least two or more of these.

More specifically, the VDAC gene expression level can be determined by measuring the mRNA which is the transcript of the VDAC gene by, for example, RNA amplification by RT-PCR (reverse transcribed-polymerase chain reaction; E. S. Kawasaki, et al., Amplification of RNA. In PCR Protocols, A Guide to Methods and Applications, Academic Press, Inc., San Diego, 21-27 (1991)), by methods of measurement on the cell level utilizing such a technique as northern blotting analysis (Molecular Cloning, Cold Spring Harbor Lab. (1989)), in situ RT-PCR (Nucl. Acids Res., 21, 3159-3166 (1991)) or in situ hybridization, or by NASBA (Nucleic acid sequence-based amplification, Nature, 350, 91-92 (1991)).

A more preferred detection method may be a RT-PCR-based detection method. This can be carried out more conveniently according to the real time PCR method (cf. e.g. Proc. Natl. Acad. Sci., 88, 7276-7280 (1991); Japanese Unexamined Patent Publication No. H06-500021; Biotechniques, 22, 176-181 (1997)) enabling the real time detection of the desired PCR amplification product.

More specifically, the RT-PCR method can be carried out in the following manner. Thus, RNA is extracted from the sample in the conventional manner, cDNA is prepared from the RNA and, using this as a template, a pair of primers (forward chain binding to the above cDNA (- chain) and reverse chain binding to the + chain) designed so as to enable amplification of the VDAC gene region, which is the target, are allowed to hybridize with the template. Then, the PCR is carried out in the conventional manner, and the amplified double-stranded DNA is detected. This amplified double-stranded DNA can be detected by the real time PCR technique or by some other method, for example, the method comprising carrying out the above PCR using primers labeled in advance with a radioisotope or a fluorescent substance, for instance, and detecting the thus-produced labeled double-stranded DNA, or the method comprising transferring the double-stranded DNA produced to a nylon membrane, for instance, in the conventional manner and detecting the product of hybridization with a labeled probe.

The primers to be used in the above method are required only to enable specific amplification of the desired VDAC and can be designed in an appropriate manner based on the VDAC DNA sequence information known in the art. It is generally recommended that the primers each have a nucleotide length of about 10-35, preferably about 15-30. Similarly, the probe is designed so as to be capable of specifically hybridizing with the PCR amplification product and enable specific detection of the amplification product. The probe may have approximately the same nucleotide length as the primers and can be labeled, for example, with an arbitrary reporter fluorescent dye and an arbitrary quencher fluorescent dye so that it may be utilized in the detection according to the real time PCR technique.

These primers and probe can be synthesized in the conventional manner using an automated DNA synthesizer, for example the DNA synthesizer Pharmacia LKB Gene Assembler Plus (product of Pharmacia).

Apparatus, instrument and general-purpose kits for carrying out the RT-PCR or real time PCR are commercially available and, in carrying out the measurements according to the present invention, those commercial products can adequately be utilized.

Various procedures employable in the above, for example RNA extraction, DNA or DNA fragment synthesis, enzymatic treatments for cleavage, deletion, addition or ligation, RNA or DNA isolation, purification, replication, selection and amplification, can all be carried out in the conventional manner (cf. e.g. Bunshi-Idengaku Jikkenho (Experiments in Molecular Genetics), published 1983 by Kyoritsu Shuppan Co., Ltd.; PCR Technology, published 1990 by Takara Shuzo Co., Ltd.). It is also possible to appropriately modify these in application thereof. Further, in determining the base sequence of the amplification product, for instance, various methods known in the art or possibly developed in the future can be widely used (e.g. cycle sequencing: cf. e.g. Shohei Hattori, "Direct base sequence determination using PCR", Jikken Igaku (Experimental Medicine) (special issue), New PCR Techniques and Applications, 29-35 (1997), published by Yodosha).

The VDAC protein expression level determination can be carried out in the conventional manner according to such a known method as western blotting or ELISA utilizing, for example, an antibody to the VDAC protein. More specifically, the western blotting, for instance, can be carried out by using an antibody against the VDAC protein as a primary antibody, then using a labeled antibody (labeled antibody capable of binding to the primary antibody) labeled with a radioisotope such as ¹²⁵I or a fluorescent substance, for instance, as a secondary antibody, and detecting and measuring the signal originating in the radioisotope or fluorescent substance of the labeled binding product obtained by means of a radiation detector (e.g. BAS-1800II: product of Fuji Photo Film Co.) or a fluorescence detector, for instance.

The physiological activity of the VDAC protein, i.e. the measurement target in the above, is already known and, reliably, there is a certain correlation between the amount of that protein and the activity thereof. Therefore, the detection (diagnosis) according to the invention can also be carried out by measuring the activity of that protein in lieu of the protein level. Thus, the present invention also includes a method of diagnosing encephalitis/encephalopathy which comprises measuring and evaluating the activity of the VDAC protein, which serves as an indicator, by any of the known methods.

In accordance with the method of diagnosis according to the invention, it is judged that there is the onset or the onset risk of encephalitis/encephalopathy when the VDAC expression level (VDAC gene expression level, VDAC protein level or VDAC activity; these are collectively referred to herein as "VDAC expression level") as measured in the manner described hereinabove shows an increase. This increase can be judged, for example, by measuring in advance the VDAC expression levels of normal subject-derived samples, determining the mean or statistical median value and using this value as a reference value for comparison, or by carrying out a plurality of measurements for each patient at timed intervals and comparing a later measured value with an earlier measured value as a reference value.

### Antibodies to the VDAC protein

Antibodies to the VDAC protein can be prepared using the VDAC protein as an immuongen. The antibodies include both antisera (polyclonal antibodies) and monoclonal antibodies. The methods of producing antibodies are themselves well understood by those skilled in the art. The antibodies to the VDAC protein can also be produced by these conventional methods (cf. e.g. Zoku Seikagaku Jikken Koza (Lectures on Biochemical Experiments, second series) "Men-eki Seikagaku Kenkyuho (Methods in Immunobiochemistry)", edited by the Japanese Biochemical Society (1986)). The thus-obtained antibodies can be advantageously utilized, for example, for purifying the VDAC protein, or assaying or identifying the same by immunological techniques. The antibodies to the VDAC protein are partly available on the market, and these commercial products can be adequately used in the practice of the invention as well.

A specific example of the VDAC expression level measurement by the antibody-based immunological means is, for example, as shown later herein in the examples section. The antibodies are thus useful in the pharmaceutical field as reagents for measuring the VDAC expression levels, and as reagents for diagnosing encephalitis/encephalopathy in which the VDAC is involved. Further, as described later herein, the antibodies are also useful in screening for candidate drugs capable of suppressing (inhibiting) the VDAC activity.

A diagnostic agent or reagent kit (detecting agent or reagent kit) for encephalitis/encephalopathy is suitably utilized in the method of diagnosing encephalitis/encephalopathy according to the invention. The kit comprises, as active ingredients, reagents appropriate as means for carrying out the above-mentioned VDAC expression level measurement.

The kit comprises, as essential components, specific reagents appropriate for the method of determining the VDAC expression level. Such specific reagents are properly selected according to the detection method to be employed. The reagents include, among others, an antibody to the VDAC protein, a probe for detecting the transcript of the VDAC gene and/or a primer set for detection, etc. Thus, the reagents are characterized as reagents necessary for the means for specifically detecting the measurement target according to the invention. The general-purpose reagents for carrying out the PCR are not considered as essential components of such diagnostic kit but, like reagents for hybridization, for instance, they may be included in the diagnostic kit. The present invention thus also provides such a reagent kit for the diagnosis of encephalitis/encephalopathy.

### (2) Method of determining the onset risk of encephalitis/encephalopathy as incurred by a drug (method of drug testing)

The present invention also provides a method of examining a known or novel drug to be utilized in the treatment of influenza infection, for instance, as to whether it incurs the risk of causing encephalitis/encephalopathy upon administration thereof or not, or a method of estimating the level of such risk, namely a method of testing the safety (safety testing) of a drug as to the onset of encephalitis/encephalopathy.

This method of drug testing is carried out by measuring the VDAC expression level in VDAC-expressing cells in a system in which a drug is present and comparing that expression level with the VDAC expression level in VDAC-expressing cells in a system in which a drug is absent, with the difference (variation) in said expression level as an indicator. If the VDAC expression level in VDAC-expressing cells in the system in which the drug is present at least shows no increase as compared with that in the system in which the drug is absent (i.e. the difference is zero or minus), the drug tested can be judged as having no risk of causing encephalitis/encephalopathy.

The above-mentioned system in which a drug is present include (a) a system formed by administration of a drug to test animals (in vivo system) and (b) a system formed by addition of a drug to a VDAC-expressing cell culture fluid (in vitro system).

The test animals are not particularly restricted but model animals in a condition of mitochondrial stress can be preferably utilized. Such model animals show an increased VDAC expression level and are in a preparatory stage for the onset of encephalitis/encephalopathy (brain edema) (increased brain edema sensitivity stage) and, when such a trigger as influenza virus infection acts on such model animals, severe edema manifests itself (influenzal encephalitis/encephalopathy onset models) and, therefore, those animals can be properly used in the above-mentioned drug testing.

The model animals under mitochondrial stress can be prepared by inducing a disorder in long-chain fatty acid metabolism in the mitochondrial inner membrane. As typical examples of the model animals, there may be mentioned, for example, carnitine transporter OCTN2-deficient mice (e.g. JVS (juvenile visceral steatosis) mice) and mice administered with a carnitine antagonist (e.g. β-(2,2,2-trimethylhydrazium)-propionate; THP).

A drug to be tested is administered to these test animals, and the VDAC expression levels in various tissues and/or cells, in particular brain tissues, of the animals are determined. In cases where an increase in that expression level (the reference being the expression level in a drug-free system resulting from omission of administration of the test drug) is observed (when the difference is plus), the test drug can be judged to have the onset risk of encephalitis/encephalopathy. When there is no increase in the expression level in question or there is a decrease in the expression level, the test drug can be judged to be safe, namely have no onset risk of encephalitis/encephalopathy.

The drug testing according to the invention can be carried out utilizing a system set up by adding the test drug to a VDAC-expressing cell culture fluid. The VDAC-expressing cells utilizable in this system are those derived from various tissues or cells carrying the VDAC or the gene therefor; thus, for example, brain cells, lung cells, liver cells, or established cell strains thereof may be used. The VDAC-expressing cells may be recombinant cells artificially caused to express the VDAC by introduction of the VDAC gene in the conventional manner. The VDAC-expressing cells further include those cells derived from model animals in the above-mentioned condition of mitochondrial stress.

The cells to be used in the test method according to the invention also include tissues, which are aggregates of cells, for example brain tissue, lung tissue and liver tissue.

These cells can be maintained in an ordinary cell culture medium, and the VDAC expression level in the cells may be measured in the conventional manner in the presence of a predetermined amount of the test drug caused in advance to exist in the medium or in the absence of the test drug. The conditions (temperature, pH, medium composition, etc.) of the test drug-containing system and test drug-free system are the same as in the conventional procedures and are to be properly selected from among the conditions under which the cells will not die but the VDAC can be expressed. In the above testing method, the VDAC expression level can be measured by such various methods as described above under (1).

### (3) Method of screening for candidate drugs for the treatment of encephalitis/encephalopathy

The invention further provides a method of screening for candidate drugs which comprises measuring the VDAC expression level in VDAC-expressing cells in a system in which a test substance is present, comparing the thus-measured expression level with the VDAC expression level in the VDAC-expressing cells in a system in which said test substance is absent (which level is taken as a reference value), and selecting the test substance as a candidate drug when it is effective in lowering the VDAC expression level as compared with the reference value (having a VDAC expression level depressing activity).

Utilizable as the test substance-containing system and test substance-free system in the above screening method are the same ones as the drug-containing system and drug-free system in the in vivo system and in vitro system described in detail above under (2). The treatment with the test substance (administration to the test animals and addition to the cell culture fluid) and the VDAC expression level measurement are carried out also in the same manner. The test substance to be subjected to this screening method is not particularly restricted but includes nucleic acids (including antisense nucleotides to the VDAC gene), peptides, proteins, organic compounds, inorganic compounds and so forth.

The screening method of the invention selects, as candidate drugs, those test substances which lower the VDAC expression level taken as an indicator. By this screening, it is possible to select potential candidate drugs for enabling the treatment for prevention, alleviation or cure of encephalitis/encephalopathy.

A preferred embodiment of the screening method of the invention comprises measuring the level of binding of miniplasmin, plasmin or plasminogen to the VDAC protein in a system in which a test substance is present, comparing the thus-measured binding level with the result (reference value) of measurement of the level of binding of miniplasmin or the like to the VDAC protein in a system in which said test substance is absent, and selecting the test substance as a candidate drug when it is effective in lowering the level of binding as compared with the reference value. This method utilizes one of the functions of the VDAC protein, namely the function thereof to allow miniplasmin, plasmin and plasminogen to bind to that VDAC protein as a receptor. A candidate substance capable of inhibiting the binding of miniplasmin and the like to the VDAC protein as a receptor is useful as a drug for the treatment of encephalitis/encephalopathy.

This method can be carried out utilizing the VDAC protein, which is known in the art, and at least one substance selected from the group consisting of miniplasmin, plasmin and plasminogen and examining the effect of the test substance on the binding between both. Thus, the method can be carried out by examining to which extent the test substance can lower (inhibit) the binding of miniplasmin, plasmin or plasminogen to the VDAC protein. The measurement of the level of binding between the VDAC protein and miniplasmin, plasmin or plasminogen can be carried out in the conventional manner, for example, utilizing known antibodies specific to the VDAC protein, miniplasmin, plasmin and plasminogen, respectively.

The candidate drugs that can be selected by the screening method of the invention include the above-mentioned nucleic acids, peptides, proteins, etc. as well as those drugs which inhibit the conversion of plasminogen having no proteolytic enzyme activity to miniplasmin or plasmin having proteolytic enzyme activity and, as a result, prevent the production of miniplasmin; plasmin binding to the VDAC protein and prevent the onset of encephalitis/encephalopathy, for example plasminogen activator inhibitors, granulocyte elastase inhibitors, cathepsin D inhibitors, etc.

### (4) Drug for the treatment of encephalitis/encephalopathy

The candidate drug selected by the screening method described above under (3) is useful as a drug for the treatment of encephalitis/encephalopathy. Therefore, the invention further provides agents for the treatment of encephalitis/encephalopathy which comprises such drug as an active ingredient.

The active ingredient in the above-mentioned treatment agent according to the invention may be either one selected by utilizing the screening method of the invention or one produced by a chemical or biochemical technique or on a commercial basis in the conventional manner based on the information about that selected drug.

The active ingredient can be used either as such or in admixture with pharmaceutically acceptable known carriers (excipients or diluents, fillers, binders, lubricants, etc.) and so forth to give pharmaceutical compositions. In cases where the active ingredient is a protein, the pharmaceutical compositions are preferably prepared by properly using various components used in ordinary protein preparations, for example stabilizers, sterilizing agents, buffer agents, isotonizing agents, chelating agents, pH adjusting agents, surfactants, etc.

The pharmaceutical compositions can be administered either orally or parenterally according to the forms in which they are prepared (oral dosage forms such as tablets, pills, capsules, powders, granules, syrups, etc.; parenteral dosage forms such as injections, drip infusion preparations, external preparations, suppositories, etc.). The pharmaceutical preparations of the invention can also take the form of storable preparations prepared by lyophilization of solution preparations; they can be used by extemporaneously dissolving in water, physiological saline or a like buffered solution to an appropriate concentration.

The pharmaceutical preparations according to the invention contain a pharmacologically effective amount of the active ingredient, and the dosage thereof may vary according to the active ingredient species, the route of administration, the age, weight and symptoms of the administration target or patient but can be properly selected by those skilled in the art based on the effects in animal tests, among others.

The present inventors have revealed that the VDAC plays an important role in the onset of encephalitis/encephalopathy, and further have demonstrated that one of the factors leading to an increased level of expression of that VDAC is a disorder in fatty acid metabolism in mitochondria. In view of this, a drug capable of restoring or improving the disordered fatty acid metabolism is thought to be able to contribute, through administration thereof, to the normalization of the VDAC expression level and thus produce the curative and ameliorating effects on encephalitis/encephalopathy. Therefore, those patients who have been given a diagnosis of the onset of encephalitis/encephalopathy in accordance with the present invention, when suspected of a disorder in fatty acid metabolism, are to be properly treated with a drug for improving that disorder. Drugs containing carnitine and/or glucose as an active ingredient are exemplified as such drug. The drugs may take the same forms as given above for the pharmaceutical compositions provided that they contain at least one active ingredient selected from among carnitine and glucose. Of course, they may be those carnitine preparations and/or glucose preparations already in use as prescription drugs.

### EXAMPLES

In the following, examples are given to illustrate the present invention in further detail.

### Example 1

### (Test materials and methods)

### (1) Test viral strain, experimental animal and reagents

- Viral strain: The non-neurotropic influenza A/Aichi/68(H3N2) strain (provided by Mr. Masato Tashiro, National Institute of Infectious Diseases) was used.
- Experimental animals: As for the wild type mice, normal C57BL/6J mice (WT) were purchased from Japan SLC, Inc. and Charles River Japan Inc. and used. As the model mice in a mitochondrial stress condition, carnitine transporter OCTN2-deficient C57BL/6J mice manifesting juvenile visceral steatosis (JVS) were used (provided by Assistant Professor Masamichi Kuwajima, University of Tokushima School of Medicine) (Kuwajima, M., et al., "Animal model of systemic carnitine deficiency: analysis in C3H-H2° strain of mouse associated with juvenile visceral steatosis". Biochem. Biophys. Res. Commun. 174, 1090-1094, 1999; Tamai, Z. et al., "Molecular and functional characterization of organic cation/carnitine transporter family in mice". J. Biol. Chem. 275, 40064-40072, 2000). New born (suckling stage) mice from day 2 after birth to the weaning stage (3 weeks of age after birth) were mainly used in the experiment as mice at an age most susceptible to influenzal encephalitis/encephalopathy.
- Antipyretics: Diclofenac sodium and aspirin (acetylsalicylic acid) (Sigma) were used.
- β-oxidation disorder inducing agent: The carnitine antagonist β-(2,2,2-triemthylhydrazium)propionate (THP) was used as a reagent temporarily and reversibly inducing disordered in vivo fatty acid β-oxidation and thus inducing an internal condition similar to that in JVS mice.

### (2) Method of infection with the virus

The influenza virus A/Aichi/68(H3N2) proliferated in embryonated chicken eggs or MDCK cells (epithelial cell strain) (given from RIKEN Cell Engineering Division) was diluted with physiological saline, and 3-day-old mice were intranasally infected, under ether anesthesia, with 4 µl of the dilution (1.2 x 10⁴ PFU). Mice in the weaning period were intranasally infected, under ether anesthesia, with 8 µl of a like dilution (3.3 x 10⁴ PFU). After virus infection, the body weights and the mortality were measured daily. In assaying virus titers, the animals were euthanized under ether anesthesia and organs were collected and used for the assay experiments.

### (3) Administration of the antipyretics and THP

A 25-µl portion of a solution of diclofenac sodium in physiological saline (1 µg/ml) or 25 µl of a 30 µg/ml solution of aspirin was subcutaneously injected into each mouse at a dorsal cervical site twice a day. The daily dose on the body weight basis was 4 mg/kg/day for diclofenac sodium or 120 mg/kg/day for aspirin. THP was dissolved in physiological saline and 25 µl of the solution was administered to each mouse twice a day at a mouse dorsal cervical site. The daily dose was 300 mg/kg/day.

### (4) Assay of brain edema with Evans Blue

Each mouse was put under ether anesthesia, the left thorax bone alone was subjected to thoractomy, and 10 µl of an Evans Blue solution (20 mg/ml of physiological saline) was injected to the mouse through the left cardiac ventricle using a microcapillary. Ten minutes later, the right atrium was incised, the Evans Blue in systemic blood vessels was removed by perfusion with an amount 6 times the body weight of physiological saline from the left cardiac ventricle, and the Evans Blue that had infiltrated into tissues was reextracted and the amount thereof was determined and used as an indicator of edema.

### (5) Quantitation of the influenza virus RNA

For detecting the influenza virus RNA in various organs such as brain, lung and liver, the total RNA was extracted from each organ using TRIzol reagent (Gibco BRL) and, using 1 µg of the total RNA, the RT-PCR (reverse transcription-polymerase chain reaction) was carried out for detecting the virus. The primers used were synthesized on an automated synthesizer. Based on the nucleotide sequence of influenza virus hemagglutinin (HA) (Verhoeyen, M., et al., "Antigenic drift between the haemagglutinin of the Hong Kong influenza strains A/Aichi/2/68 and A/Victoria/3/75". Nature 286, 771-776, 1980; Kida, H. et al., "Origin of the hemagglutinin gene of H3N2 influenza viruses from pigs in China". Virology 162, 160-166, 1988), the primers P1: SEQ ID NO:1 and P2: SEQ ID NO:2 were used in the first PCR for 578 bp detection. Further, P3: SEQ ID NO:3 and P4: SEQ ID NO:4 were used in the second PCR for 232 bp detection. In the real time PCR using a fluorescence-labeled probe, P5: SEQ ID NO:5 and P6: SEQ ID NO:6, together with the TaqMan™ probe (SEQ ID NO:7, with FAM bound to the 5' terminus and TRMRA to the 3' terminus), were used, and measurements were carried out on ABI Prism 7700 (Applied Biosystems). A working curve was prepared using a plasmid constructed by insertion of the HA cDNA of the influenza virus A/Aichi/68(H3N2) into pGEM-T Vector (Promega), and quantitations were carried out using this as a standard working curve.

### (6) Investigation regarding the increase in VDAC mRNA expression level by the RT-PCR

The brain and lung RNAs of experimental animals were extracted in the same manner as mentioned above using the TRIzol reagent (Gibco) in accordance with the manual attached thereto by the manufacturer.

### (7) Quantitation of the VDAC by the real time PCR

Using 1 µg of the RNA extracted from a tissue or cells, cDNA was prepared by the reverse transcription reaction using M-MLV Reverse Transcriptase, RNase H (Promega) and oligo(dT). Using this cDNA as a template, a 0.3 µM primer set-containing reaction mixture was prepared using QuantiTect SYBR™ Green PCT Kit (Qiagen) and, using ABI PRISM™ 7700 Sequence Detection System (Applied Biosystems), the PCR was carried out, after 15 minutes of thermal denaturation at 95°C, repeating the cycle comprising 15 seconds at 94°C, 30 seconds at 55-60°C and 30 seconds at 72°C 30-40 times, and the PCR amplification product was analyzed.

The VDAC includes three isomers, and the following primer sets (all synthesized on an automated synthesizer) were used for detecting the respective isomers (in the case of mice).
VDAC1, 335 bp amplification product
   Forward primer: SEQ ID NO:8
   Reverse primer: SEQ ID NO:9
VDAC2, 457 bp amplification product
   Forward primer: SEQ ID NO:10
   Reverse primer: SEQ ID NO:11
VDAC3, 255 bp amplification product
   Forward primer: SEQ ID NO:12
   Reverse primer: SEQ ID NO:13

For the detection of β-actin as an internal standard, the primer under SEQ ID NO:14 was used as the forward primer, and the primer under SEQ ID NO:15 as the reverse primer.

The cDNA obtained in the above RT-PCR was cloned into pGEM-T Vector (Promega), followed by isotope labeling using Prime-a-Gene™ Labeling System (Promega) for use as a probe in northern blotting.

In the above, the following primer sets for human VDAC detection can be used for the respective isomers.
VDAC1, 181 bp amplification product
   Forward primer: SEQ ID NO:16
   Reverse primer: SEQ ID NO:17
VDAC2, 486 bp amplification product
   Forward primer: SEQ ID NO:18
   Reverse primer: SEQ ID NO:19
VDAC3, 255 bp amplification product
   Forward primer: SEQ ID NO:12
   Reverse primer: SEQ ID NO:13

### (8) Detection of an influenza virus antigen, miniplasmin and the VDAC by immunohistochemical analysis

An antibody obtained by immunizing a rabbit with the influenza virus A/Aichi/68(H3N2) was purified using Protein A Sepharose™ (Amersham Biosciences). This IgG was deprived of nonspecifically reacting IgG by passage through a Sepharose™ 4B column with the mouse whole serum protein immobilized thereon, and the unadsorbed IgG fraction was used as the primary antiinfluenza virus antibody.

The primary antibody specific to miniplasmin as produced by the method of Murakami, M. et al. (Eur. J. Biochem. 268, 2847-2855, 2001) was utilized. The primary antibodies against plasmin and plasminogen utilized were the commercial products available from American Diagnostica.

Oncogene's VDAC Ab-5 was used as the primary antibody to VDAC.

Each purified specific antibody IgG (1 µg/ml) was reacted with a buffer/paraformaldehyde-immobilized brain section at 4°C overnight, followed by 1 hour of reaction with a secondary antibody at room temperature. Then, each antigen was detected according to the avidin-biotin-peroxidase complex method.

### (Study results and discussion)

### (1) Brain edema accompanying influenza infection

In mitochondria, ATP is produced in the TCA cycle mainly via acetyl coenzyme A (Acetyl-CoA), and various causes leading to decreases in acetyl coenzyme A level are known, specifically disorders in the route of long-chain fatty acid metabolism by β-oxidation via the acylcarnitine, for example impaired medium fatty acid metabolism, and disorders in the route of glucose metabolism via the acetyl coenzyme A. The above-mentioned JVS mice and THP-given mice are model mice under mitochondrial stress with a disorder in long-chain fatty acid metabolism in the mitochondrial inner membrane as induced on the cell membrane level.

As a result of the investigation of the time course of brain edema accompanying influenza infection using these JVS mice (measurement of brain edema as described above under (4) with the tissue invasion of Evans Blue taken as an indicator), the extent of brain edema increased as a function of time after influenza infection. This increase in the extent of brain edema was clearly significant as compared with the wild type mice (WT) tested in the same manner, with the Evans Blue level in the cerebral parenchyma being observed increasing with time. In the same test performed in a condition without influenza infection (in mice not infected with the virus), no brain edema was observed either in WT or in JVS mice. It has thus been clarified that the mitochondrial stress induces increased sensitivity to brain edema but by itself will not trigger brain edema and that, in this test system, influenza virus infection triggers it.

### (2) Influenza virus antigen and miniplasmin in brain

Immunohistochemical staining of the virus antigen and miniplasmin in the brain in JVS mice infected with influenza confirmed that the accumulation of miniplasmin in the cerebral blood vessel endothelium begins in several days following infection, accompanied by virus proliferation in the cerebral blood vessel endothelium. As regards this miniplasmin accumulated in the brain, it was estimated that miniplasmin is produced in large amounts at sites of inflammation in the lung and is then systemically transported in the blood flow and accumulated in the cerebral blood vessel endothelium, in particular.

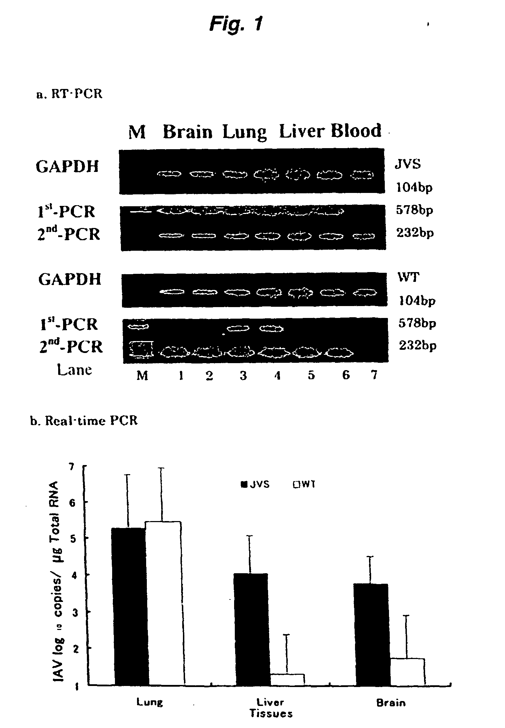

In Fig. 1 a (RT-PCR), there are comparatively shown the numbers of copies of the influenza virus genome in the brain ('Brain' in the figure), lung ('Lung' in the figure), liver ('Liver' in the figure) and blood ('Blood' in the figure) in WT and JVS mice after infection. In the figure, "1^{st}-PCR" and "2^{nd}-PCR" respectively refer to the results of the first PCR and second PCR in the above-mentioned RT-PCR, and "GAPDH" refers to the results of an internal standard. In the lanes in the figure, "M" indicates markers (DNA ladder markers).

In Fig. 1 b (Real-time PCR), there are shown comparatively the numbers of copies of the influenza virus genome in the brain ('Brain' in the figure), lung ('Lung' in the figure) and liver ('Liver' in the figure) in WT and JVS mice after infection. In the figure, the ordinate denotes the number of copies of the virus genome as measured by the above-mentioned real time PCR (on the logarithmic scale IAV log₁₀ copies/µg total RNA), and the black bars show the results in JVS mice and the white bars in WT mice.

From the results shown in Fig. 1, it was confirmed that while there was observed no great difference in number of viral replications in the lung between WT and JVS mice, the virus multiplications in the brain and liver were thousands to hundreds of times faster in JVS mice than in WT mice.

### (3) Level of binding between miniplasmin and the VDAC protein

Human plasminogen, miniplasmin and microplasmin, each in an amount of 50 ng, were dot blotted onto nitrocellulose membranes and then reacted with a 100 ng/ml purified mouse VDAC protein solution. The bound VDAC protein was detected using an anti-VDAC protein antibody (Oncogene) and an ECL chemiluminescence reagent (Amersham). The purified mouse VDAC protein solution was prepared by the method described in the literature (Bureau, M. H., et al., "Isolation and cloning of a voltage-dependent anion channel-like Mr 36,000 polypeptide from mammalian brain", J. Biol. Chem., 267, 8679-8684, 1992).

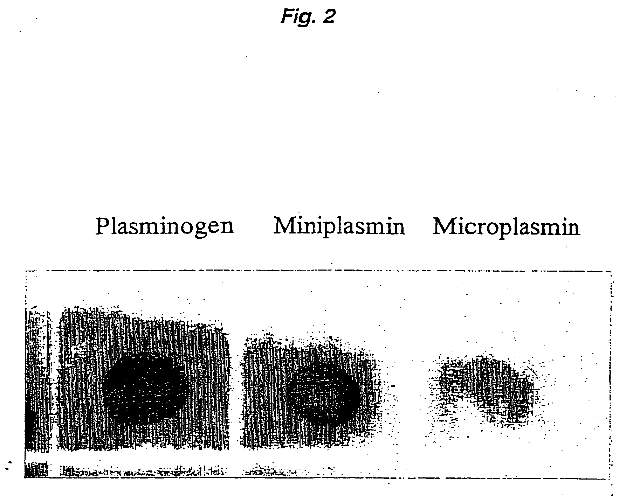

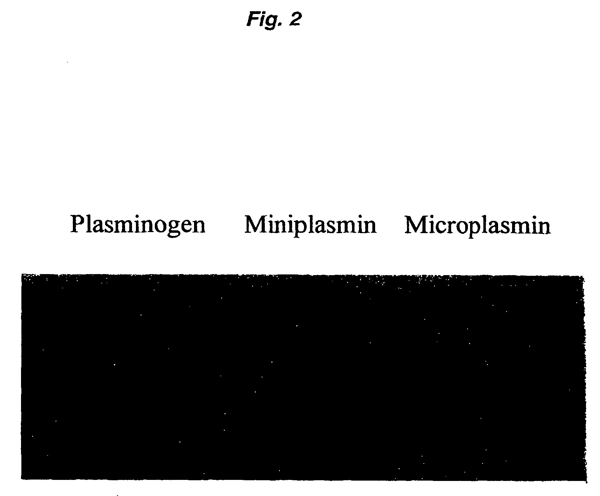

The results are shown in Fig. 2.

The results shown in Fig. 2 indicate the following. The purified VDAC protein bound to plasminogen having Kringle domains 1-5 and miniplasmin having Kringle 5 but did not bind to microplasmin having no Kringle domain. This has made it clear that at least the Kringle 5 domain is involved in the binding to the VDAC protein.

While the VDAC protein is a channel found in the mitochondrial outer membrane, the expression thereof in the cell membrane has also been confirmed. In murine brains, it was found that the VDAC protein is expressed in larger amounts on the cell membrane than in mitochondria. Thus, it was estimated that the binding between miniplasmin and the VDAC protein is realized through Kringle 5 and thus miniplasmin is accumulated in the cell membrane with the VDAC protein serving as a receptor.

### (4) VDAC expression level

There are three isomers of VDAC (VDAC-1, -2, -3). The VDAC expression in the WT mouse brain is at a relatively low level when the brain is in a normal condition. When the brain is exposed to a substance inducing influenzal encephalitis/encephalopathy, however, the VDAC expression increases and produces a condition preparatory for brain edema (condition increased in sensitivity). Specifically, brain edema is scarcely observed in that condition but, when influenza virus infection accompanies, severe edema manifests itself.

In this test, the VDAC-1 mRNA expression levels (according to the RT-PCR described above) in WT mouse brains upon treatment with various drugs and/or upon influenza virus infection were examined.

The test was carried out in the following manner: suckling WT mice were intranasally infected with the influenza virus and the VDAC-1 mRNA level was measured 5 days after infection (group a), THP, a carnitine antagonist, was administered to WT at a dose of 300 mg/kg/day for 5 days and the VDAC-1 mRNA level was then measured in the same manner (group b), the carnitine antagonist THP was administered to WT infected with influenza at a dose of 300 mg/kg/day for 5 days and then the VDAC-1 mRNA level was measured in the same manner (group c), or aspirin was administered to WT infected with influenza at a dose of 120 mg/kg/day for 5 days and then the VDAC-1 mRNA level was measured in the same manner (group d). In a control group, physiological saline was given, by injection, to WT not infected with influenza (group e). Each group consisted of 2 mice.

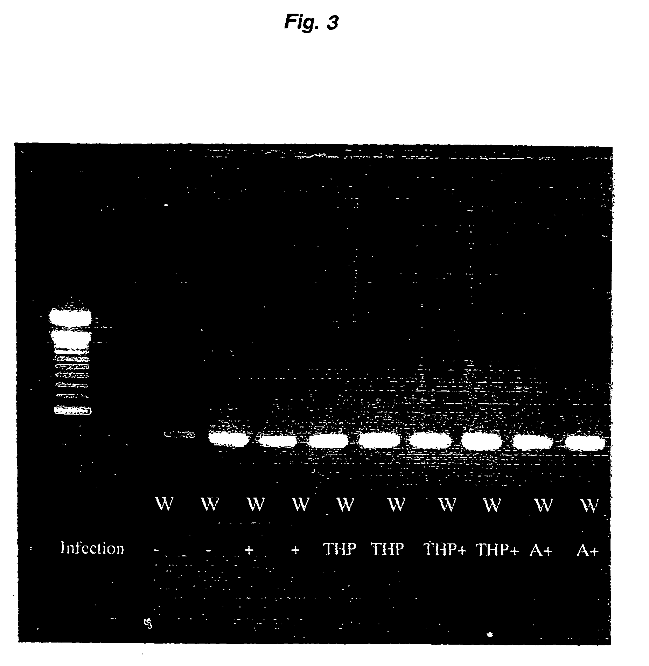

The results obtained in the above manner are shown in Fig. 3 (results of RT-PCR analysis).

In Fig. 3, W denotes the test mouse (WT). For the respective lanes, the symbols used under "Infection" have the following meanings: - (minus) denotes the group without influenza infection (control group); + (plus) denotes the group infected with the influenza virus (group a); THP denotes the group treated with THP alone (no influenza infection, group b); THP+ denotes the group of mice infected with influenza and treated with THP (group c); and A+ denotes the group of mice infected with influenza and treated with aspirin (group d).

The results shown in Fig. 3 clearly indicate the following. Thus, in group a, a distinct increase in VDAC-1 expression level in the brain was observed. In group b and group c, namely in the groups in which a mitochondrial stress condition, in particular a fatty acid metabolism disorder, was induced by treatment with the carnitine antagonist THP, significant increases in VDAC-1 were observed. In group d treated with aspirin, which is known to be a substance inducing Reye's syndrome, an increase in VDAC-1 expression level was also observed.

### (5) VDAC-1, VDAC-2 and VDAC-3 expression levels in the brain and lung

For investigating the results shown above under (4) in further detail, the VDAC-1, VDAC-2 and VDAC-3 mRNAs in the brain and lung of WT mice were assayed by the above-mentioned real time PCR in the following groups.
Group C: A control WT group given physiological saline (no influenza infection);
Group I: A WT group 5 days after influenza infection;
Group A: A WT group given 120 mg/kg/day of aspirin for 5 days;
Group A+I: A WT group give 120 mg/kg/day of aspirin for 5 days on and after the first day of influenza infection;
Group D: A WT group given 4 mg/kg/day of diclofenac sodium for 5 days;
Group THP: A WT group given 300 mg/kg/day of THP for 5 days;
Group THP+I: A WT group given 300 mg/kg/day of THP for 5 days on and after the first day of influenza infection.

Each group consisted of 5 test animals, and the results obtained are shown in terms of mean of the five animals in each group. The differences as compared with group C were tested for significance in the manner of unpaired t test using the Statview™ 4.0 software.

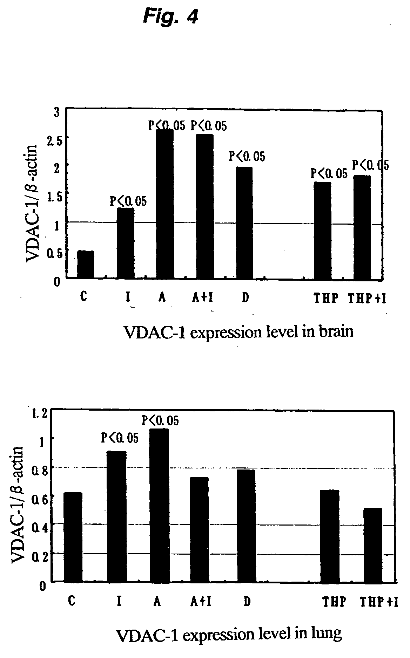

The results are shown in Figs. 4-6 in terms of relative levels of each VDAC as compared with β-actin used as an internal standard.

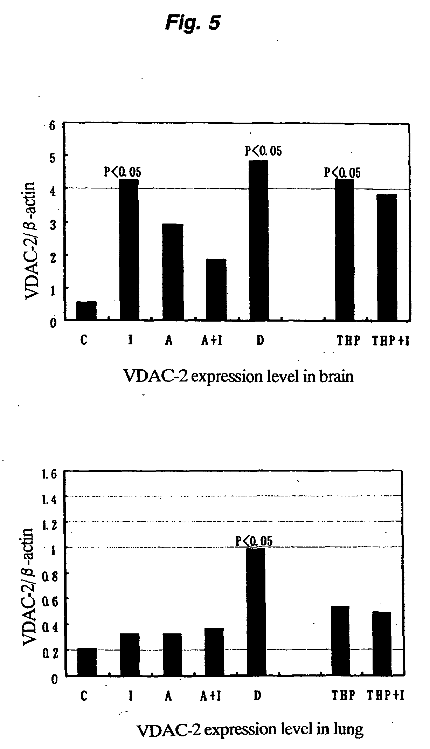

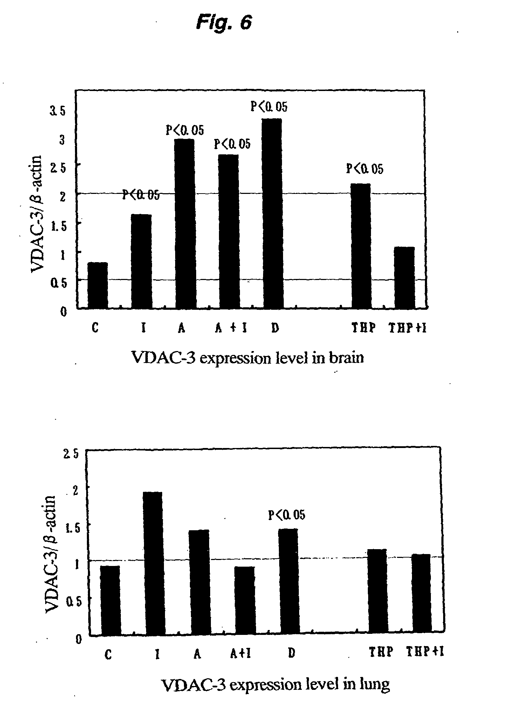

Each figure is a graphic representation of the expression levels as obtained based on the results of measurements by the real time PCR. In each figure, the upper graph shows the expression levels in the brain, and the lower graph shows the expression levels in the lung. Fig. 4 shows the results of VDAC-1 expression level measurements, Fig. 5 the results of VDAC-2 expression level measurements, and Fig. 6 the results of VDAC-3 expression level measurements.

The results shown in the respective figures indicate the following. Thus, increases in the respective VDAC mRNA expression levels were observed in the brain and lung in the influenza infection group (group I), aspirin-given group (group A), diclofenac sodium-given group (group D) and THP-given group (group THP) as compared with the physiological saline treatment (group C), although the sensitivities to the increases in expression level varied according to VDAC-1, -2 and -3, respectively.

For VDAC-1 and -2, the increases in expression level were observed in the brain in direct response to the above treatment, whereas the VDAC-3 levels responded sensitively in the lung. These facts indicate that the VDAC expression conditions in an individual, hence the expression conditions and extents in the brain (condition preparatory for brain edema) of that individual, can be estimated by studying the changes in VDAC expression in other organs or cells than brain, for example in leukocytes in cerebrospinal fluid or in peripheral blood, in human VDAC expression searching.

### (6) VDAC protein expression in the JVS mouse brain

Increases in VDAC expression in the cerebral blood vessel endothelium and nerve cells in JVS mice after intranasal influenza virus infection were checked by microscopic examination following Evans staining of the respective tissues (immunohistochemical staining).

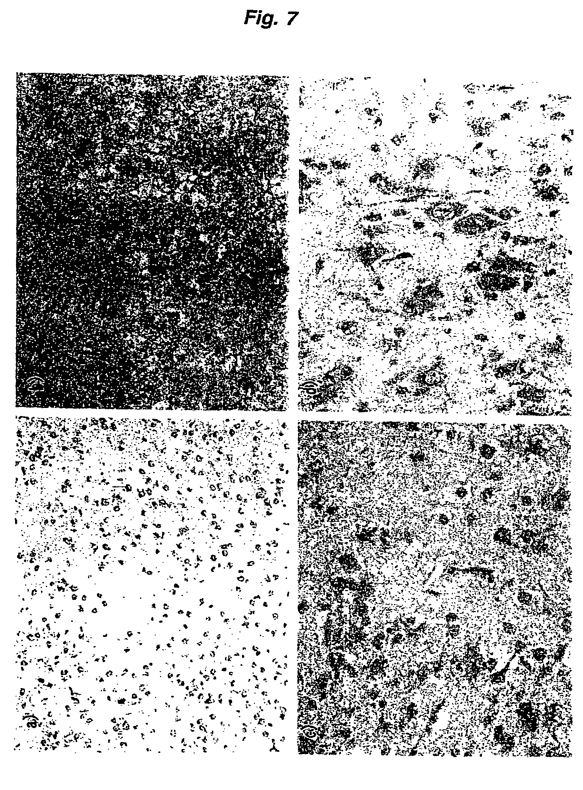

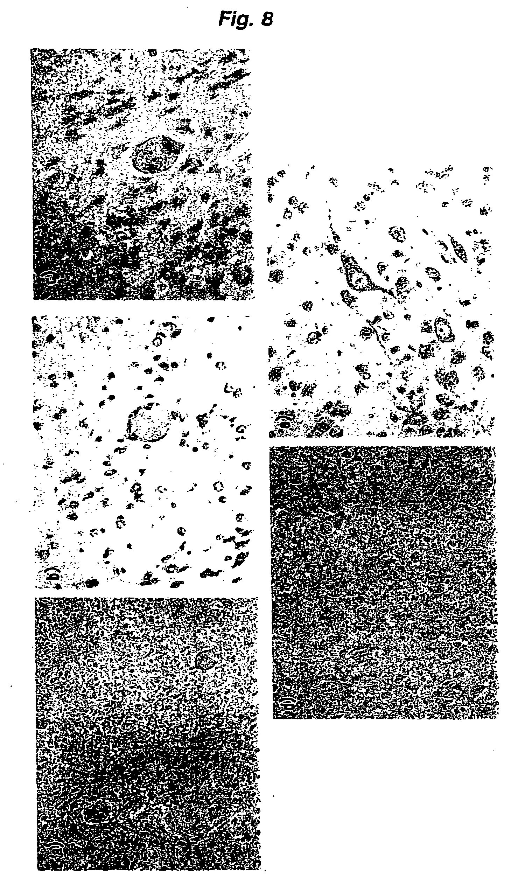

The results of photomicrography of influenza infection-free JVS mouse brains are shown in Fig. 7, and the results of photomicrography of JVS mouse brains 5 days after influenza infection in Fig. 8.

In Fig. 7, the photos a) and c) are weakly magnified ones (x 140), and b) and d) are strongly magnified photos (x 420). As shown in Fig. 7, the blood vessel endothelium was not stained, but nerve cells were stained, as shown in d).

In Fig. 8, the photos a) and d) are weakly magnified ones (x 140), and b), c) and e) are strongly magnified ones (x 420). As shown in a) to c) in that figure, the VDAC protein expression level specifically and significantly increased in the blood vessel endothelial cells after virus infection. The expression in nerve cells also increased, as seen in d) and e).

In JVS mice having disordered long-chain fatty acid metabolism in the mitochondrial inner membrane, the VDAC expression level in the brain is slightly higher as compared with WT mice. As shown in Fig. 7, the VDAC expression is observed mainly in nerve cells alone but cannot be clearly confirmed in the blood vessel endothelium. However, in the brains 5 days after intranasal influenza virus infection, significant increases in VDAC expression in the cerebral blood vessel endothelium as well as increases in nerve cells were observed, as shown in Fig. 8. Non-immunized IgG used in a control experiment completely failed to stain any of the samples, though not shown herein; thus, the proteins detected in Figs. 7 and 8 are estimated to have resulted from staining owing to a specific reaction to the VDAC antibody.

In view of the foregoing, the mechanisms of the onset of influenzal encephalitis/encephalopathy can be estimated to be as follows. Thus, various causes (antipyretics, antispasmodics, antibiotics, various pathogen infections, congenital fatty acid metabolism disorders, etc.) produce a condition preparatory for the onset of brain edema (condition highly susceptible to brain edema) and, when various infections, typically influenza virus infection or other inflammatory responses due to various causes, occur in such a condition, influenzal encephalitis/encephalopathy manifests itself.

### (7) Discussion

It was estimated that when the VDAC expression abnormally increases and a condition preparatory for brain edema (condition highly susceptible to brain edema) is thereby produced and an additional pathogen infection causes a further increase in VDAC expression or miniplasmin is produced due to an inflammatory response within the body and accumulated in the cerebral blood vessel endothelium, the blood-brain barrier is destructed or rendered unstable, leading to the onset of encephalitis/encephalopathy. As specific triggers for encephalitis/encephalopathy, there may be mentioned, among others, influenza virus infection and other virus infections, for example RS virus, adenovirus, measles virus, poliovirus, and malaria infections. In addition to infections with pathogenic microorganisms, various inflammatory responses are estimated to add to the condition highly susceptible to brain edema, leading to the onset of encephalitis/encephalopathy. Therefore, by examining the VDAC expression levels, as revealed by the present invention, it becomes possible to check the condition preparatory for the onset of brain edema (condition highly susceptible to brain edema). Further, it is important to examine the VDAC expression levels as a method of testing and judging the safety of an antipyretic, antispasmodic, antibiotic or like drug against influenzal encephalitis/encephalopathy. The method of diagnosis according to the invention is very useful as a novel method capable of diagnosing as to whether the patient is in a condition preparatory for the onset of encephalitis/encephalopathy in Reye's syndrome, influenzal encephalitis/encephalopathy, and Reye-like syndrome due to a congenital fatty acid metabolism disorder and, further, as a method capable of diagnosing beforehand the susceptibility to the onset of brain edema on the occasion of an injury or trauma or surgery.

### INDUSTRIAL APPLICABILITY

The invention provides a method of detecting the onset of encephalitis/encephalopathy and the risk thereof. This method can examine the condition preparatory for the onset of brain edema (condition highly susceptible to brain edema) by checking the VDAC expression levels. The invention further provides a method of judging as to whether the administration or application of a drug such as an antipyretic, antispasmodic, or antibiotic against influenza is safe or not (as to whether it has the risk of inducing influenzal encephalitis/encephalopathy or not). The invention further provides a method of screening for drugs utilizable in the treatment of encephalitis/encephalopathy. These methods are useful in the field of medicine as technologies of diagnosing, treating and preventing encephalitis/encephalopathy.

## Claims

1. A method of detecting encephalitis/encephalopathy comprising preparing a voltage-dependent anion channel (VDAC)-containing sample derived from a patient suspected of the onset of encephalitis/encephalopathy, measuring the level of VDAC expression in the sample, and detecting the onset of encephalitis/encephalopathy and the onset sensitivity (onset risk) using the increase in said expression level as an indicator.

2. A method of detection according to Claim 1, wherein the encephalitis/encephalopathy is influenzal encephalitis/encephalopathy.

3. A method of detection according to Claim 1, wherein the measurement of the VDAC expression level is carried out in the manner of measurement of the VDAC gene transcript.

4. A method of detection according to Claim 1, wherein the measurement of the VDAC expression level is carried out in the manner of measurement of the VDAC protein.

5. A method of detection according to Claim 1, wherein the VDAC comprises at least one isoform selected from the group consisting of VDAC-1, VDAC-2 and VDAC-3.

6. A method of determining the onset risk of encephalitis/encephalopathy as entailed by a drug which comprises measuring the VDAC expression level in VDAC-expressing cells in a system in which said drug is present, comparing said expression level with the VDAC expression level in the VDAC-expressing cells in a system in which said drug is absent, and determining the onset risk of encephalitis/encephalopathy as incurred by the drug using the difference in expression level between both as an indicator.

7. A method of determining the onset risk of encephalitis/encephalopathy as entailed by a drug according to Claim 6, wherein the system in which said drug is present is formed by administration of the drug to a test animal.

8. A method of determining the onset risk of encephalitis/encephalopathy as entailed by a drug according to Claim 6, wherein the system in which said drug is present is formed by addition of the drug to a VDAC-expressing cell culture fluid.

9. A method of screening for candidate drugs for the treatment of encephalitis/encephalopathy comprising measuring the VDAC expression level in VDAC-expressing cells in a system in which a test substance is present, comparing the thus-measured expression level with the VDAC expression level in the VDAC-expressing cells in a system in which said test substance is absent, which level serves as a reference value, and selecting the test substance as a candidate drug when it is effective in lowering the expression level as compared with the reference value.

10. A method of screening for candidate drugs capable of inhibiting the binding of miniplasmin, plasmin or plasminogen to the VDAC as a receptor, which method comprises measuring the level of binding of miniplasmin, plasmin or plasminogen to the VDAC protein in a system in which a test substance is present, comparing the thus-measured binding level with the level of binding of miniplasmin, plasmin or plasminogen to the VDAC protein in a system in which said test substance is absent, which level serves as a reference value, and selecting the test substance as a candidate drug when it is effective in lowering the level of binding as compared with the reference value.


SEQUENCE LISTING
<110> HIROSHI KIDO
<120> A method of determining the risk of developing encephalitis/encephal
opathy
<130> P04-14
<150> JP 2003-51678
<151> 2003-02-27
<160> 19
<170> PatentIn Ver. 2.1

<210> 1
<211> 20
<212> DNA
<213> Artificial Sequence
<220>
<223> Description of Artificial Sequence: primer sequence P1 (influenza A
virus Hemagglutinine)
<400> 1
tgaagtgact aatgctactg 20

<210> 2
<211> 20
<212> DNA
<213> Artificial Sequence
<220>
<223> Description of Artificial Sequence: primer sequence P2 (influenza A
virus Hemagglutinine)
<400> 2
acagacccct tacccagggt 20

<210> 3
<211> 20
<212> DNA
<213> Artificial Sequence
<220>
<223> Description of Artificial Sequence: primer sequence P3 (influenza A
virus Hemagglutinine)
<400> 3
gcaactgtta cccttatgat                                                    20

<210> 4
<211> 20
<212> DNA
<213> Artificial Sequence
<220>
<223> Description of Artificial Sequence: primer sequence P4 (influenza A
virus Hemagglutinine)
<400> 4
tcattgtttg gcatagtcac                                                    20

<210> 5
<211> 18
<212> DNA
<213> Artificial Sequence
<220>
<223> Description of Artificial Sequence: primer sequence P5
<400> 5
tctggctctc ggccaaga                                                      18

<210> 6
<211> 18
<212> DNA
<213> Artificial Sequence
<220>
<223> Description of Artificial Sequence: primer sequence P6
<400> 6
ttggcaccgc atgatgtc                                                    18

<210> 7
<211> 24
<212> DNA
<213> Artificial Sequence
<220>
<223> Description of Artificial Sequence: Taq man probe
<400> 7
ttccaggaaatga caacagcaca gcaa                                          24

<210> 8
<211> 24
<212> DNA
<213> Artificial Sequence
<220>
<223> Description of Artificial Sequence: primer sequence VDAC1-Foward
<400> 8
gctaaggatg actcggcttt aagg                                             24

<210> 9

<211> 24

<212> DNA

<213> Artificial Sequence

<220>

<223> Description of Artificial Sequence: primer sequence VDAC1-Reverse

<400> 9

aggttaagtg atgggctagg atgg                                                24

<210> 10

<211> 24

<212> DNA

<213> Artificial Sequence

<220>

<223> Description of Artificial Sequence: primer sequence VDAC2-Foward

<400> 10

tcactgttgg ctggttccta gttg                                                24

<210> 11

<211> 24

<212> DNA

<213> Artificial Sequence

<220>

<223> Description of Artificial Sequence: primer sequence VDAC2-Reverse

<400> 11

aagacctcgt ggattatgct aggg                                                24

<210> 12

<211> 23

<212> DNA

<213> Artificial Sequence

<220>

<223> Description of Artificial Sequence: primer sequence VDAC3-Foward

<400> 12

cacttgtccc tggaaatgaa gag                                                23

<210> 13

<211> 24

<212> DNA

<213> Artificial Sequence

<220>

<223> Description of Artificial Sequence: primer sequence VDAC3-Reverse

<400> 13

catgacacta cgttgttgct gagg                                               24

<210> 14

<211> 23

<212> DNA

<213> Artificial Sequence

<220>

<223> Description of Artificial Sequence: primer sequence beta-actin Forwa
rd

<400> 14

tcactattgg caacgagcgg ttc                                                23

<210> 15

<211> 23

<212> DNA

<213> Artificial Sequence

<220>
<223> Description of Artificial Sequence: primer sequence beta-actin Reves
e
<400> 15
tgtgttggca tagaggtctt tac                                              23

<210> 16
<211> 20
<212> DNA
<213> Artificial Sequence
<220>
<223> Description of Artificial Sequence: primer sequence human VDAC1-Fowa
rd
<400> 16
gccaagtatc agattgaccc                                                  20

<210> 17
<211> 22
<212> DNA
<213> Artificial Sequence
<220>
<223> Description of Artificial Sequence: primer sequence human VDAC1-Reve
rse
<400> 17
cttgaaattc cagtcctaga cc                                               22

<210> 18
<211> 21
<212> DNA

<213> Artificial Sequence
<220>
<223> Description of Artificial Sequence: primer sequence human VDAC2-Fowa
rd
<400> 18
ctaccttctc accaaacaca g                                                21

<210> 19
<211> 20
<212> DNA
<213> Artificial Sequence
<220>
<223> Description of Artificial Sequence: primer sequence human VDAC2-Reve
rse
<400> 19
ccagagcaga gagtgtaagc                                                  20